(19) Europäisches Patentamt
European Patent Office
Office européen des brevets

(11) EP 3 612 153 B1

(12) **EUROPEAN PATENT SPECIFICATION**

(45) Date of publication and mention of the grant of the patent:
**13.10.2021 Bulletin 2021/41**

(21) Application number: **18714784.8**

(22) Date of filing: **05.04.2018**

(51) Int Cl.:
***A61K 8/36*** *(2006.01)* ***A61K 8/41*** *(2006.01)*
***A61Q 5/00*** *(2006.01)* ***A61Q 5/02*** *(2006.01)*
***A61Q 5/10*** *(2006.01)* ***A61Q 17/00*** *(2006.01)*
***A61Q 19/10*** *(2006.01)* ***A61Q 5/12*** *(2006.01)*
***A61P 17/10*** *(2006.01)* ***A61K 31/133*** *(2006.01)*
***A61K 31/201*** *(2006.01)* ***A61K 8/27*** *(2006.01)*
***A61K 8/49*** *(2006.01)*

(86) International application number:
**PCT/EP2018/058668**

(87) International publication number:
**WO 2018/192777 (25.10.2018 Gazette 2018/43)**

(54) **ANTIMICROBIAL COMPOSITION FOR TREATING DANDRUFF**
ANTIMIKROBIELLE ZUSAMMENSETZUNG ZUR BEHANDLUNG VON SCHUPPEN
COMPOSITION ANTIMICROBIENNE POUR LE TRAITEMENT DES PELLICULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.04.2017 PCT/CN2017/081285**
**30.05.2017 EP 17173371**

(43) Date of publication of application:
**26.02.2020 Bulletin 2020/09**

(73) Proprietors:
- **Unilever Global IP Limited**
**Wirral, CH62 AZD (GB)**
Designated Contracting States:
**CY DE GB IE IT MT**
- **Unilever IP Holdings B.V.**
**3013 AL Rotterdam (NL)**
Designated Contracting States:
**AL AT BE BG CH CZ DK EE ES FI FR GR HR HU IS LI LT LU LV MC MK NL NO PL PT RO RS SE SI SK SM TR**

(72) Inventors:
- **CHU, Chung-Ching**
**Shanghai 200335 (CN)**
- **PU, Mingming**
**Shanghai 200335 (CN)**

(74) Representative: **Tansley, Sally Elizabeth**
**Unilever N.V.**
**Unilever Patent Group**
**Bronland 14**
**6708 WH Wageningen (NL)**

(56) References cited:
**EP-A1- 2 497 481** **WO-A1-2010/080543**
**US-A- 6 110 908** **US-A1- 2013 303 503**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 612 153 B1

## Description

### Field of the invention

This invention relates to an antimicrobial composition. The invention more particularly relates to a personal care composition e.g. those for care of hair, which provides anti-microbial efficacy. It more particularly relates to a cleansing composition for hair and scalp comprising actives that interact to provide synergistic antimicrobial efficacy for anti-dandruff benefits.

### Background of the invention

The invention relates to an anti-microbial composition useful for cleaning of any body part but especially suitable for hair and scalp. Hair care compositions generally provide cleansing or conditioning benefits or a combination of the two. Such compositions typically comprise one or more cleansing surfactants which generally aid in cleaning the hair and/or the scalp free of undesirable soil, particles, and fatty matter.

Additionally, anti-dandruff benefit has been provided through hair care compositions. Dandruff is an issue that affects many people globally. The condition is manifested by the shedding of clumps of dead skin cells from the scalp. These are white in colour and provide an aesthetically displeasing appearance. A factor that contributes to dandruff are certain members of the Malassezia yeasts. To combat these, anti-dandruff products have been developed in the form of hair cleansing shampoos. An example of a known anti-dandruff shampoo comprises sodium lauryl ether sulfate (an ethoxylated anionic surfactant) in combination with an anti-dandruff agent. Typical anti-dandruff agents used in hair care are metal pyrithione e.g. zinc pyrithione (ZPTO), octopirox® (piroctone olamine), azole antimicrobials (e.g. climbazole), selenium sulfide and combinations thereof.

While the problem of dandruff is mitigated to a large extent through use of the above actives in such compositions, there is a need for enhancing the efficacy of these actives. The present inventors have found that the efficacy of one of the above actives (zinc pyrithione) can be enhanced when combined with one or more of an antimicrobial lipid (AML). AMLs are generally present naturally in sebum which is secreted from sebaceous glands on the skin or scalp. AMLs are also derived from skin keratinocytes and carried into the stratum corneum at the skin surface. The various AMLs claimed in the present invention are generally naturally present in about 0 to 2 micrograms per $cm^2$ on the skin or scalp. The antimicrobial lipids found to be especially useful in combination with ZPTO in the present invention are sapienic acid, palmitoleic acid, sphingosine, dihydrosphingosine, and phytosphingosine. The inventors further found that other anti-microbial agents useful for combating dandruff e.g. climbazole or other skin care actives like niacinamide do not provide the synergy along with AMLs as ZPTO does.

The present inventors have found that this combination of actives is also useful in synergistically enhancing the antimicrobial efficacy against microbes like S. aureus.

US5882665A (Elizabeth Arden, 1999) discloses a novel series of phytosphingosines which are phytosphingosine salicylates having use in cosmetic compositions. They have potential as anti-acne, anti-blemish, anti-bacterial and anti-wrinkle agents, as well as being active in skin lightening.

US6110908 (Guthery B Eugene, 2000) discloses an antiseptic formulation for topical application to animal skin comprising an antimicrobial alcohol selected from ethanol, isopropanol, n-propanol and mixtures thereof; an antimicrobial lipid selected from the group consisting of free fatty acids having from six to eighteen carbons; glycerol monostearate and mixtures thereof; and zinc pyrithione.

In the present invention the antimicrobial lipids claimed are in no way disclosed in US6110908 and further the activity that can be obtained from present invention does not depend on antimicrobial alcohols which are very well known potent antimicrobials. It is said that usually such antiseptic compositions are not persistent, i.e., long-lasting in their performance. Further, it is disclosed that antimicrobial lipids, especially free fatty acids and fatty acid esters also contribute to the broad spectrum and fast acting activity of the alcohols and act synergistically with alcohols to achieve broad spectrum, fast acting antimicrobial activity. It is further disclosed that zinc omadine, like zinc pyrithione also contributes towards persistence.It is further disclosed that stable emulsion containing zinc omadine, alcohol and fatty acids is obtained when appropriate amount of ethoxylated cetyl and stearyl alcohol are included. Otherwise the composition is unstable.

WO2010080543 A1 (Guthery B Eugene) discloses a 2-step skin treatment regimen for acne vulgaris. two distinct formulations are sequentially applied to the affected area as disclosed herein: a first skin wash formulation and a second leave-on formulation, each of which comprise zinc pyrithione but in different concentrations. In a preferred leave-on composition, other anti-acne ingredients are employed along with ZPT and reference is made to one or more fatty acids or fatty acid esters selected from unsaturated fatty acids.

US20130303503 A1 (P&G) discloses that commercialized soap products claim to incorporate 2% of zinc pyrithione as an active ingredient for treating chronic skin conditions, such as dandruff, scaling, flaking, itchiness, and redness, but the detected zinc pyrithione level is substantially lower than claimed. This indicates that a majority of the

zinc pyrithione oxidizes and no longer effective as a biocidial agent. At high pH level, zinc pyrithione becomes highly susceptible to oxidation. The problem is solved by formulating a bar of soap whose pH is higher than about 9.6 and lower than about 10.3 when dispersed in an aqueous solution at 1 wt %.

EP2497481 A1 (ELC Management LLC) discloses leave-on skin care cosmetic compositions. A few exemplary formulations contain 0.1 wt% ZPTO and 0.1 to 0.2 wt% phytosphingosine. However, the presence of these ingredients in the formulations has no relation with the invention. Therefore, these ingredients seem to have been included as standard additional ingredients without expecting any unexpected interaction between the two.

To our knowledge the AMLs claimed in the present invention in combination with ZPTO have not been disclosed for synergistic antimicrobial activity.

It is thus an object of the present invention to provide for a personal care composition that exhibits synergistic antimicrobial activity as compared to the individual components.

## Summary of the invention

According to the first aspect of the present invention there is a non-therapeutic method of preventing or alleviating the symptoms of dandruff on the scalp and/or hair comprising the step of applying an anti-microbial composition to scalp and/or hair, followed by the step of rinsing off the composition from the surface after said application, where said composition comprises:

(i) 0.1 to 3% by weight of zinc pyrithione;
(ii) 0.01 to 5.0% by weight of an antimicrobial lipid selected from sapienic acid, palmitoleic acid, sphingosine, dihydrosphingosine, and phytosphingosine; and
(iii) a cosmetically acceptable vehicle, wherein said composition is a shampoo or a conditioner for preventing or alleviating the symptoms of dandruff on

the scalp and/or hair and where an antimicrobial alcohol having 1 to 7 carbon atoms are absent from the composition.

In a second aspect is disclosed non-therapeutic use of a composition for preventing or alleviating the symptoms of dandruff on the scalp and/or hair, where said composition comprises:

(i) 0.1 to 3% by weight of zinc pyrithione;
(ii) 0.01 to 5.0% by weight of an antimicrobial lipid selected from sapienic acid, palmitoleic acid, sphingosine, dihydrosphingosine, and phytosphingosine; and
(iii) a cosmetically acceptable vehicle, wherein said composition is a shampoo or a conditioner for preventing or alleviating the symptoms of dandruff on the scalp and/or hair and where an antimicrobial alcohol having 1 to 7 carbon atoms are absent from the composition.

In a third aspect is disclosed an anti-microbial composition for use in a method for preventing or alleviating the symptoms of dandruff on the scalp and/or hair, said composition comprising:

(i) 0.1 to 3% by weight of zinc pyrithione;
(ii) 0.01 to 5.0% by weight of an antimicrobial lipid selected from sapienic acid, palmitoleic acid, sphingosine, dihydrosphingosine, and phytosphingosine; and
(iii) a cosmetically acceptable vehicle,

wherein said composition is a shampoo or a conditioner and where an antimicrobial alcohol having 1 to 7 carbon atoms are absent from the composition.

## Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed

in the format "from x to y" are understood to include x and y. When for a specific feature multiple preferred ranges are described in the format "from x to y", it is understood that all ranges combining the different endpoints are also contemplated. In other words, in specifying any ranges of values, any particular upper value can be associated with any particular lower value.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

By 'an antimicrobial composition' as used herein, is meant to include a composition for topical application to hair and/or scalp of mammals, especially humans. Such a composition is generally applied on to the desired topical surface of the body for a period of time from a few seconds to up to 24 hours. When the period of time of application is low say of the order of a few seconds to a few minutes after which the composition is rinsed off with water or wiped away, such a composition is known as a cleansing composition or a wash-off composition. On the other hand, When the composition is applied for longer period of time say from several minutes to up to 24 hours and washed off usually during the process of normal personal cleaning, such a composition is known as a leave-on composition. The composition is of the wash-off type. It includes any product applied to a human body for also improving appearance, cleansing, odor control or general aesthetics. The composition is a shampoo or a conditioner. It is used for preventing or alleviating the symptoms of dandruff on the scalp and/or hair.

The composition is an anti-dandruff hair care composition. It comprises synergistic anti-fungal action of the polyvalent metal salt of pyrithione with one or more of the antimicrobial lipid compounds claimed in the present invention.

The polyvalent metal salt of pyrithione is zinc pyrithione (ZPTO) which is shorthand for zinc 1-hydroxy-2-pyridinethione.

The polyvalent metal salt of pyrithione is represented by the following general formula:

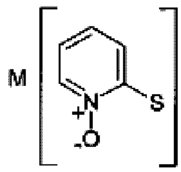

In the case of zinc pyrithione, M is the metal cation zinc.

Zinc pyrithione is present in 0.1 to 3.0%, more preferably from 0.1 to 2.0% based on weight of the composition. ZPTO is a particulate material. While the particle size is not critical to achieve the benefits of the present invention, the particle size of ZPTO is preferably from 0.25 to 8 micrometer, more preferably from 0.5 to 8.0 micrometer, and further more preferably from 1.0 to 7.5 micrometer. ZPTO is commercially available from Kolon Life Science Inc., Sino Lion (USA) Ltd, Lonza, and other suppliers.

The antimicrobial lipids which in combination with zinc pyrithione provide the synergistic antimicrobial activity include sapienic acid, sphingosine, dihydrosphingosine, palmitoleic acid, phytosphingosine or combinations of one of more of the AMLs. More preferred AMLs are sapienic acid and sphingosine most preferred being sphingosine.

Sapienic acid and palmitoleic acid are generally added into the antimicrobial agents in purified form. These fatty acids may be chemically synthesized or naturally sourced and can be in free fatty acid form or in esterified form, preferably free form. Natural substances which contain these fatty acids and its derivatives may come from animal or plant sources, including but not limited to plant seed extract, marine oils, animal oil and microbial ferments. Specifically, sapienic acid was reported as a major constituent of the seed oil of Thunbergia alata (85% by weight as the triglyceride). Purified sapienic acid or seed oil extract comprising sapienic acid may be added to the antimicrobial composition, while ensuring that sapienic acid is present in the desired concentration in the composition.

Sphingosine, phytosphingosine, and dihydrosphingosine, are sphingoid bases present on the human skin. They can be synthesized chemically or produced via suitable biotechnological process that lead to formation of sphingoid bases by yeast from materials like sugar. Sphingoid bases may be added to the antimicrobial composition in purified form. Alternatively, fermentation broth consisting of high quantities of sphingoid bases may be used, while ensuring that sphingoid bases are present in the desired concentration in the composition.

The AMLs are included in 0.01 to 5% by weight of the composition, preferably 0.1 to 2%. It is preferred that the weight ratio of zinc pyrithione to the AMLs that are present is in the range of 8:1 to 1:1500 at the cellular level for the antimicrobial action to be most effective.

Evonik Industries is a major supplier of AMLs including sphingosine, phytosphingosine, cholesterol, and fatty

acids.

Without wishing to be bound by theory, the inventors believe that the anti-microbial efficacy of ZPTO is enhanced by the AML through microbial membrane disruption.

In the present invention, antimicrobial alcohols (low molecular weight alcohols having one to 7 carbon atoms) are absent. By absent is meant that the concentration of the alcohol is less than an amount that is necessary for antimicrobial therpeutic activity. Preferably, the antimicrobial alcohol is present in less than 1%, more preferably less than 0.1%, based on total weight of the composition.

The composition comprises a cosmetically acceptable vehicle. According to one aspect the cosmetically acceptable carrier comprises water. According to another preferred aspect, the carrier additionally comprises a surfactant. The cosmetically acceptable vehicle is such that the composition can be prepared as a shampoo, or conditioner.

As per an aspect of the invention, the composition is a shampoo. The composition especially shampoos are formulated with an anionic surfactant e.g. an alkyl sulphate and/or ethoxylated alkyl sulfate surfactant. These anionic surfactants are preferably present at a level of from 1 to 20%, preferably 2 to 16%, furthermore preferably from 3 to 16% by weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium.

Preferred alkyl ether sulfates are those having the formula: $RO(CH_2CH_2O)_nSO_3M$; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES). SLES having an average degree of ethoxylation of from 0.5 to 3, preferably 1 to 3 is especially preferred.

The shampoo compositions may comprise one or more further anionic cleansing surfactants which are cosmetically acceptable and suitable for topical application to the hair.

Examples of further suitable anionic cleansing surfactants are the alkaryl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in the compositions include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate.

Suitable preferred additional anionic cleansing surfactants are sodium lauryl ether sulphosuccinate(n)EO, (where n is from 1 to 3), lauryl ether carboxylic acid (n) EO (where n is from 10 to 20).

Mixtures of any of the foregoing anionic cleansing surfactants may also be suitable.

The composition preferably additionally comprises an amphoteric surfactant preferably a betaine surfactant preferably an alkyl amidopropyl betaine surfactant for example cocamidopropyl betaine. In a preferred embodiment, the composition comprises from 0.1 to 10 wt.%, preferably from 0.5 to 8 wt.%, more preferably from 1 to 5 wt.% of a betaine surfactant.

To enhance deposition of actives from the compositions especially shampoos, cationic polymers are generally included therein. In the present invention too, it is preferred that the composition additionally includes 0.01 to 2.0% of a cationic polymer. The cationic polymer is preferably guar hydroxypropyl trimonium chloride. Guar polymer predominantly contains galactomannan polymer chains. This polymer is available at various molecular weights and degree of cationic substitutions depending on how much the guar has been hydrolysed and cationised. The cationic polymer is preferably present in 0.04 to 0.5%, more preferably 0.08 to 0.25% by weight of the composition.

The pH of the composition is preferably equal to or higher than 4.0, more preferably in the range of 5.0 to 10.0.

The composition especially for anti-dandruff shampoos preferably additionally comprises a zinc compound. The presence of additional zinc compound in the composition is believed to improve the antidandruff efficacy of the metal salt of pyrithione. Suitable zinc compounds are zinc oxide, zinc citrate, zinc malonate, zinc carbonate or combinations thereof. The zinc compound is preferably present in 0.1 to 3%, more preferably 0.1 to 1.5% by weight of the composition.

The shampoo composition preferably additionally comprises a conazole fungicide.

Preferably the conazole fungicide is selected form ketoconazole, climbazole or mixtures thereof. The azole fungicide is preferably included in 0.01 to 2%, more preferably 0.025 to 0.75% by weight of the composition. The presence of a conazole fungicide is believed to improve the deposition of zinc pyrithione.

The composition preferably additionally comprises a vitamin B3 compound. The preferred vitamin B3 compound is niacinamide.

Niacinamide has the structure as given below:

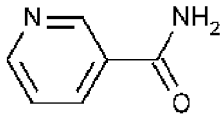

Niacinamide is known for secretion of AMPs from keratinocytes. The AMPs thus secreted provides for improving the immunity of the external surface of the body e.g. on the scalp. Thus with the use of niacinamide in the composition the anti-dandruff efficacy is expected to be enhanced not just through anti-fungal activity of the composition of the invention but by providing a boost to the scalp's own protection shield against germs, through use of niacinamide. It is expected that this combination could provide further long-lasting protection e.g. up to 24 hours of protection against germs.
Niacinamide is preferably present in 0.1 to 5%, more preferably 0.5 to 5%, furthermore preferably 0.5 to 3%, and optimally 1.0 to 3.0% by weight of the composition.

Suspending Agent

Preferably the composition further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.
Suitable cross-linked polymers of acrylic acid and acrylate esters are Pemulen® TR1 or Pemulen® TR2. A suitable heteropolysaccharide gum is xanthan gum, for example that available as Kelzan® mu.
Mixtures of any of the above suspending agents may be used. Preferred is a mixture of cross-linked polymer of acrylic acid and crystalline long chain acyl derivative.
Suspending agent, if included, will generally be present in the composition at levels of from 0.1 to 10%, preferably from 0.5 to 6%, more preferably from 0.5 to 4% by total weight of suspending agent based on the total weight of the composition.
The composition may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.
The composition is preferably aqueous based. It preferably comprises high amounts of water preferably from 70 to 95% by weight of the composition.

Hair Conditioner

When conditioning benefits are to be delivered through the composition, the composition is called a hair conditioner. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. An especially useful conditioning agent is a silicone compound, preferably a non-volatile silicone compound. Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).
Amounts of the silicone in compositions where present may range from about 0.1 to about 10 wt.%, preferably from about 0.1 to about 8wt.%, more preferably from about 0.3 to about 5wt.% by weight of the hair care compositions.
The pH of the composition is preferably equal to or higher than 4.0, more preferably in the range of 5.0 to 7.0.
The hair conditioning composition usually comprises conditioning surfactants selected from cationic surfactants,

used singly or in admixture. Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners is behenyltrimethylammonium chloride, available commercially, for example as GENAMIN® KDMP, ex Clariant. Yet another preferred cationic surfactant is stearamidopropyl dimethylamine.

The most preferred cationic surfactants for use in the composition are stearamidopropyl dimethylamine, behentrimonium chloride, or stearyl trimethyl ammonium chloride. In the conditioners, the level of cationic surfactant will generally range from 0.1 % to 5%, preferably 0.5 to 2.5% by weight of the composition.

Hair conditioning compositions preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of the compositions.

The level of fatty alcohol in the conditioners will generally range from 0.5 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, more preferably from 1:1.5 to 1:8, optimally from 1:2 to 1:5.

Water is a preferred carrier. When water is present, it is preferably present in at least 1%, more preferably at least 2%, furthermore preferably at least 5% by weight of the composition. When water is the carrier, a preferred liquid composition comprises 10 to 99.8% by weight water.

The invention will now be illustrated with reference to the following non-limiting Examples.

**Examples**

Example 1 to 5

Synergistic interaction between ZPTO and the AML compounds was determined using the ΣFIC assay against *Malassezia furfur.* A brief description of the method is given below.

Method: ΣFIC assay against *Malassezia furfur*

Microorganism preparation:

*M. furfur* (CBS 1878) was maintained on MD agar plate (Solution A) was cultured into 20ml of growth medium Pityrosporum Broth (PB, Solution B).

Solution A preparation

| | | |
|---|---|---|
| Modified Dixon Agar (MD) | | |
| 36g | Malt Extract (Oxoid) | |
| 6g | Mycological Peptone (Oxoid) | |
| 10 | Purified Agar (Oxoid) | |
| 20g | Ox-bile (Oxoid) | |
| 2ml | Oleic acid (Sigma) | |
| 2ml | Glycerol (Sigma) | |
| 10ml | Tween 40 (Sigma) | |
| Deionized Water to 1000ml | | |
| 50mg (1 vial) dissolved in 2ml 95% ethanol | | Chloramphenicol (Oxoid SR078E) |

(continued)

(Ensure stirring thoroughly including after autoclaving)

| Solution B preparation | |
| --- | --- |
| Pityrosporum Broth (PB) | |
| 10g | Bacteriological Peptone |
| 0.1g | Yeast extract |
| 10g | Ox-bile |
| 2.5g | Taurocholic acid |
| 10g | Glucose |
| 1L | Deionised water |
| 0.5ml | Tween60 |
| 1ml | Glycerol |
| Adjust pH to 6.2 | |
| After sterilization | |
| 0.5ml | UHT milk |

They are then incubated at 32 °C for 48 hours with shaking. Then, 1ml of the first broth culture is transferred into 9ml of fresh PB and incubated at 32 °C for 48 hours with shaking. The final culture should contain 2 to $6\times10^6$ cells/ml. This is achieved by diluting using PB to $5*10^5$ cell/ml.

In-vitro susceptibility testing

Zinc pyrithione was serially diluted (2-fold) to prepare in a range of 2 to 125 ppm in the growth medium. The AML compound was serially diluted (2-fold) to prepare in a range of 48.8-50,000 ppm for sapienic acid, and palmitoleic acid; and 10 to 10,000 ppm for other AMLs in growth medium.

Binary combinations of ZPTO and the AML compound were prepared in 96-well plate by mixing 20 μl of zinc pyrithione solution with 20 μl of AML compound. The solution in each well were further mixed with 160 μl of PB and 20 μl of *M. furfur*. The final cell density in the testing plate is around $5*10^4$ cell/ml, and the final concentration of actives in each well of the 96-wel plate were shown below.

ZPTO concentrations (in ppm): 12.5, 6.3, 3.1, 1.6, 0.8, 0.4, 0.2 and 0.

The fatty acid AML sapienic acid and palmitoleic acid compound concentrations (in ppm): 5000, 2500, 1250, 625, 312.5, 56.3, 78.1, 39.1, 19.5, 9.8, 4.9, and 0.

The sphingosine AML compound concentrations (in ppm): 1000, 500, 250, 125, 62.5, 31.3, 15.6, 7.8, 3.9, 2.0, 1.0 and 0.

The plate was incubated at 32°C for one day. Then 20 μl of alamar blue (0.01%) was added into each well with a further incubation for one day to observe the color change. Red color indicates microbial growth and the non-changing blue color indicates no growth. In addition, it was also confirmed that the testing compound alone will not cause color change of alamar blue with a control mixture without adding microbes.

Calculation:

(1) Minimum Inhibition Concentration (MIC):

The MIC is defined as the absolute lowest concentration of active that provides complete microbial growth inhibition as indicated by the blue color of alamar blue under the tested condition. For example, for zinc pyrithione, the MIC was determined to be 1.6-3.2 ppm.

The MIC of sapienic acid, palmitoleic acid, phytosphingosine, sphingosine and dihydrosphingosine was determined to be 2550 to 5000 ppm, 5000 ppm, 125 ppm, 1000 ppm and 1000 ppm respectively.

(2) Fractional Inhibition Concentration (FIC):

The differing behaviour of inhibitory antimicrobials in isolation and mixtures have been widely explored using the concept of the Fractional Concentration (FC) and Fractional Inhibitory Concentration (FIC). The parameter can be

defined as follows:

$$FIC\ (component\ a) = \frac{MIC\ (component\ a\ tested\ in\ the\ mixture)}{MIC\ (component\ a\ tested\ as\ a\ single\ active)}$$

(3) Synergy and Additivity

The interactions between antimicrobials can be additive, synergistic or antagonistic depending on whether the efficacy of the combination is equivalent to, greater than or less than that obtained for the same total concentration of the individual components when tested alone.

These relationships can be expressed mathematically by summing the fractional MIC values for all the components present in the mixture to give the "fractional inhibition index". There is no consistent approach in the academic or patent literature in defining precise limiting ΣFIC values that differentiate synergy from additivity or antagonism. In this study, we have adopted a liberal approach defining any binary mixture with ΣFIC < 0.9 as showing evidence of synergistic behavior.

$$\sum FIC = FIC\ (component\ 1) + FIC\ (component\ 2)$$

ΣFIC = 1 corresponds to additive microbial activity

ΣFIC > 1 corresponds to antagonistic microbial activity
ΣFIC < 0.9 corresponds to synergistic microbial activity

4. Results:

Based on the concentration range of ZPTO (0 to 12.5 ppm) and the AML compound (0 to 5000 ppm) at which the experiments were carried out, the ΣFIC of the combinations is given below in Table - 1.

Table - 1

| Example | Combination | ΣFIC |
|---|---|---|
| 1 | ZPTO + Sapienic acid | 0.75 |
| 2 | ZPTO + sphingosine | 0.50 |
| 3 | ZPTO + dihydrosphingosine | 0.75 |
| 4 | ZPTO + palmitoleic acid | 0.38 |
| 5 | ZPTO + phytosphingosine | 0.53 |

The data in Table above indicates that each of the antimicrobial lipids of the present invention in combination with ZPTO exhibit synergistic antifungal activity.

Example 6 to 10: Antibacterial efficacy of the combinations claimed in the present invention was determined_using the ΣFIC assay against *Staphylococcus aureus.* Effect of sphingosine with triclosan as well as triclocarban was also tested. A brief description of the method is given below.

Methods: ΣFIC assay against *Staphylococcus aureus*

Microorganism preparation:

S. *aureus* (ATCC 12600) was streaked on Tryptone Soya Agar plate (TSA, Solution C) and a single colony was cultured into 20ml of growth medium Tryptone Soya Broth (TSB, Solution D).

Solution C preparation (Tryptone Soya Agar, TSA)

| | |
|---|---|
| 40g | Tryptone Soya Agar (Oxoid) |
| Deionized Water to 1000 ml | |

(continued)

Autoclave

Solution D preparation (Tryptone Soya Broth, TSB)

| | |
|---|---|
| 30g | Tryptone Soya Broth (Oxoid) |
| Deionized Water to 1000ml | |
| Autoclave | |

They are then incubated at 37 °C overnight (16 hours) with shaking. The overnight culture is diluted using TSB to $5*10^4$ cell/ml.

In-vitro susceptibility testing

Zinc pyrithione was serially diluted (2-fold) to prepare in a range of 0.5 to 500 ppm in the growth medium. The AML compound was serially diluted (2-fold) to prepare in a range of 16 to 1,000 ppm for sapienic acid, and palmitoleic acid; and 4 to 250 ppm for other AMLs in growth medium. Triclosan was serially diluted (2-fold) to prepare in a range of 0.2 to 200 ppm and triclocarban in a range of 2 to 2000 ppm in growth medium.

Binary combinations of ZPTO or triclosan/triclocarban and the AML compound were prepared in 96-well plate by mixing 20 μl of zinc pyrithione solution with 20 μl of AML compound. The solution in each well were further mixed with 160 μl of TSB and 20 μl of S. *aureus.* The final cell density in the testing plate is around $4*10^4$ cell/ml, and the final concentration of actives in each well of the 96-wel plate were shown below.

ZPTO concentrations (in ppm): 50, 25, 12.5, 6.3, 3.1, 1.6, 0.8, 0.4, 0.2, 0.1, 0.05 and 0

The fatty acid AML sapienic acid and palmitoleic acid compound concentrations (in ppm): 100, 50, 25, 12.5, 6.3, 3.1, 1.6 and 0.

The sphingosine AML compound concentrations (in ppm): 25, 12.5, 6.3, 3.1, 1.6, 0.8, 0.4 and 0.

Triclosan concentrations (in ppm): 20, 10, 5, 2.5, 1.25, 0.63, 0.31, 0.16, 0.08, 0.04, 0.02 and 0.

Triclocarban concentrations (in ppm): 200, 100, 50, 25, 12.5, 6.25, 3.13, 1.56, 0.78, 0.39, 0.20 and 0.

Then 20 μl of alamar blue (0.01%) was added into each well, and the plate was incubated at 37°C for one day to observe the color change. Red color indicates microbial growth and the non-changing blue color indicates no growth. In addition, it was also confirmed that the testing compound alone will not cause color change of alamar blue with a control mixture without adding microbes.

Calculation:

(1) Minimum Inhibition Concentration (MIC):

The MIC is defined as the absolute lowest concentration of active that provides complete microbial growth inhibition as indicated by the blue color of alamar blue under the tested condition. For example, for zinc pyrithione, the MIC was determined to be 3.2 to 6.3 ppm. The MIC of triclosan and triclocarban was determined to be 0.16 ppm and 3.13 ppm.

The MIC of sapienic acid, sphingosine and dihydrosphingosine was determined to be 25 ppm, 3.1 ppm and 6.3 ppm respectively.

(2) Fractional Inhibition Concentration (FIC):

The differing behaviors of inhibitory antimicrobials in isolation and mixtures have been widely explored using the concept of the Fractional Concentration (FC) and Fractional Inhibitory Concentration (FIC). The parameter can be defined as follows:

$$\text{FIC (component a)} = \frac{\text{MIC (component a tested in the mixture)}}{\text{MIC (component a tested as a single active)}}$$

(3) Synergy and Additivity

The interactions between antimicrobials can be additive, synergistic or antagonistic depending on whether the efficacy of the combination is equivalent to, greater than or less than that obtained for the same total concentration of the individual components when tested alone.

These relationships can be expressed mathematically by summing the fractional MIC values for all the components present in the mixture to give the "fractional inhibition index". There is no consistent approach in the academic or patent literature in defining precise limiting ΣFIC values that differentiate synergy from additivity or antagonism. In this study, we have adopted a liberal approach defining any binary mixture with ΣFIC < 0.9 as showing evidence of synergistic behavior.

$$\sum FIC = FIC\ (component\ 1) + FIC\ (component\ 2)$$

ΣFIC = 1 corresponds to additive microbial activity

ΣFIC > 1 corresponds to antagonistic microbial activity
ΣFIC < 0.9 corresponds to synergistic microbial activity

4. Results:

Based on the concentration range of ZPTO (0 to 50 ppm) and the AML compound (0 to 100 ppm) at which the experiments were carried out, the ΣFIC of the combinations is given below in Table 2 below.

Table - 2

| Example | Combination | ΣFIC |
|---|---|---|
| 6 | ZPTO + Sapienic acid | 0.60 |
| 7 | ZPTO + sphingosine | 0.63 |
| 8 | ZPTO + dihydrosphingosine | 0.75 |
| 9 | Triclocarban + phytosphingosine | 1 |
| 10 | Triclosan + phytosphingosine | 1 |

The data in Table 2 above indicates that the combinations are also effective in synergistically inhibiting growth of bacteria like S. *aureus.* On the other hand, antibacterial agents such as triclosan or triclocarban only showed additive but not synergistic effect combining with AML.

It is to be understood that the experiments described above were conducted in an invitro assay to evaluate the synergistic antimicrobial behaviour. It is expected that the concentrations to be actually used to prepare a composition for topical use would be vastly different. The concentrations could be orders of magnitude higher due to the following reasons that affect the difference in concentration in the bulk as compared to that at the cellular level. The composition may be formulated as an emulsion or a gel with very many additional ingredients which affect the concentration of the desired actives in the oil phase and in the water phase which could be very different. They may also have very different physical and hydrodynamic properties like partition coefficients, diffusional rates, convective transport rates, rheological properties etc.

Therefore it is expected that the concentrations to be used when formulated as a composition would be very different from that at the cellular level, at which the experiments were carried out, usually orders of magnitude higher.

## Claims

1. A non-therapeutic method of preventing or alleviating the symptoms of dandruff on the scalp and/or hair comprising the step of applying an antimicrobial composition to scalp and/or hair, followed by the step of rinsing off the composition from the surface after said application, where said composition comprises:

   (i) 0.1 to 3% by weight of zinc pyrithione;

(ii) 0.01 to 5.0% by weight of an antimicrobial lipid selected from sapienic acid, palmitoleic acid, sphingosine, dihydrosphingosine, and phytosphingosine; and
(iii) a cosmetically acceptable vehicle, wherein said composition is a shampoo or a conditioner for preventing or alleviating the symptoms of dandruff on the scalp and/or hair and where an antimicrobial alcohol having 1 to 7 carbon atoms are absent from the composition.

**2.** A method as claimed in claim 1 wherein said composition comprises 0.1 to 2.0% zinc pyrithione.

**3.** A method as claimed in claim 1 or 2 wherein said antimicrobial composition comprises 0.1 to 2% by weight antimicrobial lipid.

**4.** A method as claimed in any of claims 1 to 3 wherein said antimicrobial composition additionally comprises a surfactant.

**5.** Non-therapeutic use of a composition for preventing or alleviating the symptoms of dandruff on the scalp and/or hair, where said composition comprises:

(i) 0.1 to 3% by weight of zinc pyrithione;
(ii) 0.01 to 5.0% by weight of an antimicrobial lipid selected from sapienic acid, palmitoleic acid, sphingosine, dihydrosphingosine, and phytosphingosine; and
(iii) a cosmetically acceptable vehicle, wherein said composition is a shampoo or a conditioner for preventing or alleviating the symptoms of dandruff on the scalp and/or hair and where an antimicrobial alcohol having 1 to 7 carbon atoms are absent from the composition.

**6.** An anti-microbial composition for use in a method for preventing or alleviating the symptoms of dandruff on the scalp and/or hair, said composition comprising:

(i) 0.1 to 3% by weight of zinc pyrithione;
(ii) 0.01 to 5.0% by weight of an antimicrobial lipid selected from sapienic acid, palmitoleic acid, sphingosine, dihydrosphingosine, and phytosphingosine; and
(iii) a cosmetically acceptable vehicle,
wherein said composition is a shampoo or a conditioner and where an antimicrobial alcohol having 1 to 7 carbon atoms are absent from the composition.

## Patentansprüche

**1.** Nicht-therapeutisches Verfahren zur Vorbeugung oder Linderung der Symptome von Schuppen auf der Kopfhaut und/oder dem Haar, umfassend den Schritt des Auftragens einer antimikrobiellen Zusammensetzung auf die Kopfhaut und/oder das Haar, gefolgt von dem Schritt des Abspülens der Zusammensetzung von der Oberfläche nach dem Auftragen, wobei die Zusammensetzung umfasst:

(i) 0,1 bis 3 Gewichts-% Zinkpyrithion;
(ii) 0,01 bis 5,0 Gewichts-% eines antimikrobiellen Lipids, ausgewählt aus Sapiensäure, Palmitoleinsäure, Sphingosin, Dihydrosphingosin und Phytosphingosin; und
(iii) ein kosmetisch akzeptables Vehikel, wobei die Zusammensetzung ein Shampoo oder eine Spülung zur Vorbeugung oder Linderung der Symptome von Schuppen auf der Kopfhaut und/oder dem Haar ist und wobei ein antimikrobieller Alkohol mit 1 bis 7 Kohlenstoffatomen in der Zusammensetzung abwesend ist.

**2.** Verfahren wie in Anspruch 1 beansprucht, wobei die Zusammensetzung 0,1 bis 2,0 % Zinkpyrithion umfasst.

**3.** Verfahren wie in Anspruch 1 oder 2 beansprucht, wobei die antimikrobielle Zusammensetzung 0,1 bis 2 Gewichts-% antimikrobielles Lipid umfasst.

**4.** Verfahren wie in irgendeinem der Ansprüche 1 bis 3 beansprucht, wobei die antimikrobielle Zusammensetzung zusätzlich ein Tensid umfasst.

**5.** Nicht-therapeutische Verwendung einer Zusammensetzung zur Vorbeugung oder Linderung der Symptome von Schuppen auf der Kopfhaut und/oder dem Haar, wobei die Zusammensetzung umfasst:

(i) 0,1 bis 3 Gewichts-% Zinkpyrithion;
(ii) 0,01 bis 5,0 Gewichts-% eines antimikrobiellen Lipids, ausgewählt aus Sapiensäure, Palmitoleinsäure, Sphingosin, Dihydrosphingosin und Phytosphingosin; und
(iii) ein kosmetisch akzeptables Vehikel, wobei die Zusammensetzung ein Shampoo oder eine Spülung zur Vorbeugung oder Linderung der Symptome von Schuppen auf der Kopfhaut und/oder dem Haar ist und wobei ein antimikrobieller Alkohol mit 1 bis 7 Kohlenstoffatomen in der Zusammensetzung abwesend ist.

**6.** Antimikrobielle Zusammensetzung zur Verwendung in einem Verfahren zur Vorbeugung oder Linderung der Symptome von Schuppen auf der Kopfhaut und/oder dem Haar, wobei die Zusammensetzung umfasst:

(i) 0,1 bis 3 Gewichts-% Zinkpyrithion;
(ii) 0,01 bis 5,0 Gewichts-% eines antimikrobiellen Lipids, ausgewählt aus Sapiensäure, Palmitoleinsäure, Sphingosin, Dihydrosphingosin und Phytosphingosin; und
(iii) ein kosmetisch akzeptables Vehikel,
wobei die Zusammensetzung ein Shampoo oder eine Spülung ist und wobei ein antimikrobieller Alkohol mit 1 bis 7 Kohlenstoffatomen in der Zusammensetzung abwesend ist.

## Revendications

**1.** Procédé non thérapeutique pour prévenir ou alléger les symptômes de pellicules sur le cuir chevelu et/ou les cheveux, comprenant l'étape d'application d'une composition antimicrobienne sur le cuir chevelu et/ou les cheveux, suivie de l'étape d'élimination de la composition sur la surface par rinçage après ladite application, dans lequel ladite composition comprend :

(i) 0,1 à 3 % en poids de pyrithione de zinc ;
(ii) 0,01 à 5,0 % en poids d'un lipide antimicrobien choisi parmi l'acide sapiénique, l'acide palmitoléique, la sphingosine, la dihydrosphingosine, et la phytosphingosine ; et
(iii) un véhicule acceptable en cosmétique,

dans lequel ladite composition est un shampooing ou un après-shampoing pour prévenir ou alléger les symptômes de pellicules sur le cuir chevelu et/ou les cheveux et où un alcool antimicrobien ayant 1 à 7 atomes de carbone est absent de la composition.

**2.** Procédé selon la revendication 1, dans lequel ladite composition comprend 0,1 à 2,0 % de pyrithione de zinc.

**3.** Procédé selon la revendication 1 ou 2, dans lequel ladite composition antimicrobienne comprend 0,1 à 2 % en poids de lipide antimicrobien.

**4.** Procédé selon l'une quelconque des revendications 1 à 3, dans lequel ladite composition antimicrobienne comprend de plus un tensioactif.

**5.** Utilisation non thérapeutique d'une composition pour prévenir ou alléger les symptômes de pellicules sur le cuir chevelu et/ou les cheveux, où ladite composition comprend :

(i) 0,1 à 3 % en poids de pyrithione de zinc ;
(ii) 0,01 à 5,0 % en poids d'un lipide antimicrobien choisi parmi l'acide sapiénique, l'acide palmitoléique, la sphingosine, la dihydrosphingosine, et la phytosphingosine ; et
(iii) un véhicule acceptable en cosmétique,

dans laquelle ladite composition est un shampooing ou un après-shampoing pour prévenir ou alléger les symptômes de pellicules sur le cuir chevelu et/ou les cheveux et où un alcool antimicrobien ayant 1 à 7 atomes de carbone est absent de la composition.

**6.** Composition antimicrobienne pour une utilisation dans un procédé pour prévenir ou alléger les symptômes de pellicules sur le cuir chevelu et/ou les cheveux, ladite composition comprenant :

(i) 0,1 à 3 % en poids de pyrithione de zinc ;

(ii) 0,01 à 5,0 % en poids d'un lipide antimicrobien choisi parmi l'acide sapiénique, l'acide palmitoléique, la sphingosine, la dihydrosphingosine, et la phytosphingosine ; et
(iii) un véhicule acceptable en cosmétique,

laquelle composition est un shampooing ou un après-shampoing pour prévenir ou alléger les symptômes de pellicules sur le cuir chevelu et/ou les cheveux et où un alcool antimicrobien ayant 1 à 7 atomes de carbone est absent de la composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5882665 A, Elizabeth Arden
- US 6110908 A, Guthery B Eugene
- WO 2010080543 A1, Guthery B Eugene
- US 20130303503 A1
- EP 2497481 A1